(19) Europäisches Patentamt
European Patent Office
Office européen des brevets

(11) Publication number: 0 386 691 A2

# (12) EUROPEAN PATENT APPLICATION

(21) Application number: 90104249.9

(22) Date of filing: 06.03.90

(51) Int. Cl.[5]: C12Q 1/68, C12Q 1/66, C12Q 1/32

(30) Priority: 08.03.89 JP 53902/89

(43) Date of publication of application: 12.09.90 Bulletin 90/37

(84) Designated Contracting States: DE FR GB

(71) Applicant: Toyo Boseki Kabushiki Kaisha
No.2-8, Dojimahama 2-chome Kita-ku
Osaka-shi Osaka 530(JP)

(72) Inventor: Watanabe, Haruo
Sunshine B-607, 3-10, Ohtoyamachi
Taihaku-ku, Sendai-shi, Miyagi 982(JP)
Inventor: Shibata, Shuji
1-B-301, Katata 2-chome
Ohtsu-shi, Shiga 520-02(JP)

(74) Representative: von Kreisler, Alek, Dipl.-Chem. et al
Patentanwälte Von Kreisler-Selting-Werner
Deichmannhaus am Hauptbahnhof
D-5000 Köln 1(DE)

## (54) DNA probe detection method with bacterial luciferase system.

(57) A DNA probe assay method with a bacterial luciferase system, characterized in that a DNA probe labeled with alkaline phosphatase is reacted with $NADP^+$ and the resulting $NAD^+$ is reacted with alcohol and alcohol dehydrogenase to convert it to NADH, which is then measured as a luminescence intensity using bacterium-derived flavin reductase and luciferase is disclosed. The method of the present invention is excellent in that it surpasses the absorption photometric method in sensitivity and stability because it combines a DNA probe labeled with alkaline phosphatase with a luminescent system.

EP 0 386 691 A2

# DNA PROBE DETECTION METHOD WITH BACTERIAL LUCIFERASE SYSTEM

## BACKGROUND OF THE INVENTION

The present invention relates to a DNA probe detection method for DNA probing, which utilizes nucleic acid hybridization to identify a particular base sequence.

The use of an enzyme-labeled DNA probe to identify a particular sequence of nucleic acid has recently been widely examined as a DNA probe detection method to replace the method using radioisotope as a marker. Particularly, detection of the subject substance by enzyme labeling has been widely applied as an enzymoimmunoassay method for immunological reactions. Detection systems with sensitivities comparable to, or even higher than those of radioisotopes have already been developed. Among the marker enzymes, alkaline phosphatase is expected to offer high activities because of its great number of turnovers (specific activity serves as an index). Actually, alkaline phosphatase is often selected for a marker system for this reason as well as because it remains stable while being used for labeling. This enzyme is also suitable not only for detection methods based on absorption photometry but also for those based on fluorometry because any substance can serve as a reaction substrate for alkaline phosphatase, as long as it has an external phosphoric acid group. For reduced blank values for the detection system and improved detection sensitivities, however, it is essential to choose a stable substrate which is not spontaneously decomposable at pH values near to optimum reaction conditions for the enzyme.

Also, it is of course advantageous to use the luminescence method to improve the sensitivity of the detection system for high sensitivity detection of alkaline phosphatase activity and in turn the enzyme-labeled substance. On a related note, it is theoretically possible to realize a high sensitivity system by liberating phosphoric acid from a phosphoric acid binding type luminescent or fluorescent substance (e.g. umbelliferylphosphoric acid) and causing chemiluminescence of the resulting fluorescent substance in the presence of a diester of oxalic acid, a peroxide ($H_2O_2$) and an oxidization catalyst (e.g. peroxidase). However, this method necessitates the use of a stable phosphorylated luminescent or fluoroscent substance which is not spontaneously hydrolyzable. Improvement in detection sensitivity in chemiluminescence is such that a sensitivity equal to, or at most about 10 fold higher than that obtained in fluorometry is realized.

## SUMMARY OF THE INVENTION

With this background, the present inventors made intensive investigations to develop a detection method based on luminescence, using a detection system with a DNA probe labeled with alkaline phosphatase as a marker, which permits detection with higher sensitivites.

In nucleic acid hybridization, the marker enzyme is handled at high temperatures near 60°C for the purpose of nonspecific adsorption and hybridization accuracy and efficiency, and a very reactive surfactant (e.g. SDS) is used for washing. As a marker enzyme for the DNA probe in this nucleic acid hybridization, alkaline phosphatase with high activity which endures such severe hybridization conditions is used. The present inventors found a method with high sensitivity by combining a detection system for the enzyme, with a high sensitivity luminescence system in consideration of the properties of this highly active alkaline phosphatase and the severe hybridization conditions, and developed the present invention.

The present invention provides a DNA probe assay method with a bacterial luciferase system, characterized in that a DNA probe labeled with alkaline phosphatase is reacted with $NADP^+$ and the resulting $NAD^+$ is reacted with alcohol and alcohol dehydrogenase to convert it to NADH, which is then measured as a luminescence intensity using bacterium-derived flavin reductase and luciferase.

## DETAILED DESCRIPTION OF THE INVENTION

Accordingly, the present invention comprises a DNA probe detection method which permits high sensitivity detection based on bioluminescence to improve the detection sensitivity. First, a substance must be chosen as the substrate for which alkaline phosphatase has a small K m value and which shows no considerable spontaneous hydrolysis (dephosphorylation) under substrate reaction conditions, since the

substrate needs to be for alkaline phospatase, its reaction product must be convertible to a substrate for bioluminescence, specifically for a luminescence enzyme of luminescent bacteria, and the substrate must have a very low blank value in luminescence detection, to maintain a satisfactory precision in luminescence detection. The present inventors made investigations and found that $NADP^+$ (nicotinamide adenine dinucleotide phosphate) is favorable because it meets the above-mentioned requirements. Literature [Leon A. Heppel et al.: J. Biol. Chem., vol. 237, pp. 841-846 (1961)] indicates that $NADP^+$ is a substrate for alkaline phosphatase and its hydrolysis produces $NAD^+$ (nicotinamide adenine dinucleotide). Further investigation revealed that the Km value of alkaline phosphatase to $NADP^+$ is lower than that of p-nitrophenylphosphoric acid, a substrate suitable to absorption photometry. The use of a substrate with a low Km value makes it possible to obtain a maximum activity with a small amount of substrate for alkaline phosphatase reaction; the use of a small amount of substrate reduces the amount of $NAD^+$ produced by nonenzymatic reaction, thus permitting minimization of blank value increase caused by the substrate during luminescence reaction. As an enzyme capable of selectively reducing the resulting $NAD^+$ alone in the presence of $NADP^+$ in excess in converting it to NADH, a substrate for the bacterial lumines cence system, yeast-derived alcohol dehydrogenase (EC 1.1.1.1) (e.g. enzyme produced by Boheringer Manheim) is known to specifically act on $NAD^+$. In addition, this alcohol dehydrogenase is a very advantageous enzyme because it acts under alkaline conditions in the presence of ethyl alcohol, a substrate therefor, while the necessary ethyl alcohol concentration does not hamper the alkaline phosphatase activity at all. It is therefore possible to sequentially convert the $NAD^+$ produced by the action of alkaline phosphatase to NADH by coupling with alcohol dehydrogenase in a single reaction system. Moreover, this alcohol dehydrogenase is advantageous for prevention of nonspecific reaction because it contains only very small amounts of phosphatase and $NAD^+$ decomposition enzyme (NADase). Thus, it is possible to measure the amount of the alkaline phosphatase, the amount of the enzyme-labeled DNA probe and in turn the amount of the nucleic acid sequence hybridized with the DNA probe by reacting the NADH produced and accumulated with flavin reductase and luciferase both obtained from luminescent bacteria in the presence of FMN (flavin mononucleotide), a long-chain aldehyde and oxygen, and quickly measuring the luminescence intensity as a peak height in a short time, for example, about 7 seconds. The alcohol dehydrogenase described above is not necessarily derived from yeast, as long as it possesses characters equivalent to those of yeast-derived alcohol dehydrogenase. For example, alcohol dehydrogenase derived from equine liver can be used. Note that examples of the above-mentioned luminescent bacteria include *Vibrio harveyi* and *Vibrio fischeri* (ATCC 7744). The above-mentioned luminescent bacteria are taxonomically described in "*Current Microbiology*", vol. 4, p. 127 (1980). A method of obtaining luciferase from the luminescent bacteria is described in, for example, Journal of Biological Chemistry, vol. 247, pp. 398-404 (1972). A method of obtaining flavin reductase is described in, for example, Molecular & Cellular Biochemistry, vol. 44, pp. 181-187 (1982). None of the flavin reductase and luciferase used for luminescence detection contain phosphatase, alcohol dehydrogenase or any component which causes NADH decomposition reaction; therefore, the amount of NADH could be accurately detected as an amount of luminescence. Moreover, another major advantage of the present invention is that none of the alcohol dehydrogenase, flavin reductase and luciferase reagents used for the coupling reaction are not affected by the residual surfactant.

The reaction described above can be schematized in the following reaction formulae:

$$NADP^+ \longrightarrow NAD^+ + Ⓟ$$

$$NAD^+ + C_2H_5OH \xrightarrow{\text{Alcohol dehydrogenase}} NADH + H^+ + CH_3CHO$$

$$NADH + H^+ + FMN \xrightarrow{\text{NADH-dependent flavin reductase}} FMNH_2 + NAD^+$$

$$FMNH_2 + RCHO + O_2 \xrightarrow{\text{Luciferase}} FMN + C\text{-}RCOOH + H_2O + h\nu$$

wherein $h\nu$ represents the amount of light. (In the above formula, RCHO represents a long-chain aldehyde, e.g. decanal; RCOOH represents a long-chain carboxylic acid, e.g. decanoic acid.)

In the detection method of the present invention, the target DNA is denaturated and immobilized in a single strand form on a nylon membrane and hybridized with DNA probe labeled with alkaline phosphatase, and then it is used as a sample. A known amount of the target DNA is previously immobilized and hybridized as above to draw a working curve, based on which an unknown amount of the target DNA in the sample can be determined.

The present invention is hereinafter described in more detail by means of the following examples, but the invention is not by any means limited by these examples.

## Example 1

A plasmid incorporating an HSV-generic specific sequence was prepared as a control plasmid and diluted with distilled water to obtain series of dilutions. To each dilution, an equal amount of 0.6N NaOH was added. After being kept standing at room temperature for 15 minutes, this mixture was subjected to dot blotting in an amount of 75 ul. The dot-blotted nylon membrane was gently rinsed with a buffer (5 x SSC) prepared by 4-fold diluting 20 x SSC (175 g of NaCl and 88 g of sodium citrate per liter), and then dried at 80° C for 30 minutes. This membrane was cut into a piece of 3 mm in diameter according to the dot, and this was followed by addition of a hybridization buffer (prepared by adding 5 g of BSA, 5 g of polyvinylpyrrolidone and 10 g of SDS to 20 x SSC) and heating at 60° C for 15 minutes. Then, an alkaline phosphatase-labeled DNA probe was added in an amount of 5 ng per ml hybridization buffer, and this was followed by heating at 60° C for 15 more minutes. Then, the mixture was washed with 1 x SSC (1/20 dilution) and 1% SDS at room temperature in two cycles of shaking for 5 minutes. Then, the mixture was washed with a washing solution prepared by adding 1% Triton X-100 to 1 x SSC at 60° C for 5 minutes. After cooling to room temperature, the mixture was further washed twice with the same buffer as above, and finally washed twice with 1 x SSC at room temperature for 5 minutes. The starting sample amount was 12.3 ng ($1.4 \times 10^9$ copies). The sample was used in dilutions.

A disc hybridized with the labeled DNA probe thus prepared in accordance with the series of sample DNA dilutions was placed in a cylindrical container. Then, a 20 mM tris-HCl buffer, pH 8.5, containing 0.5 mM $MgCl_2$ supplemented with 25 $\mu$l of 2 u/ml alcohol dehydrogenase, 21 mM ethyl alcohol and 100 $\mu$M $NADP^+$, was added, and this was followed by reaction at 25° C for 1 hour. After reaction, a 20 $\mu$l portion was subjected to luminescence reaction as the sample. The lumi nescence reaction reagent was a solution of 0.006% decanal, 10 $\mu$M FMN, 0.6% BSA, 50 mu/ml flavin reductase and $2 \times 10^{-2}$ mu/ml luciferase in a 0.1M phosphate buffer, pH 7.0. This luminescence reaction reagent (200 $\mu$l) was injected to the 20 $\mu$l sample; the peak height was measured. Luminescence was measured using the TD4000 Lumiphotometer produced by Laboscience Company. The measurement results are shown in Table 1. As seen in the table, the minimum sensitivity was $2.13 \times 10^4$ copies, which corresponded to 0.188 pg of plasmid. Note that the S/N ratio was 1.18, relative to the blank with a significant difference. Also, the present method proved an excellent detection system because the S/N ratio on each measuring point was very high in the concentration range in which detection is possible by absorption photometry as well.

## Comparison Example 1

A sample of plasmid incorporating the same HSV-generic specific sequence as in Example 1 was assayed by the formazan coloring method using the same series of dilutions as in Example 1 and the SNAP kit produced by MBI Company. As a result, $5.46 \times 10^6$ copies were detected (Table 1). The S/N ratio was lower than that obtained with the luminescence method. The detection system was based on the method in which the product from dephosphorylation of 5-bromo-4-chloro-3-indolyl phosphate as the substrate with

alkaline phosphatase acts nonenzymatically to reduce nitroblue tetrazolium and develop its color, this formazan pigment deposits on the membrane, and the tone of the developed color is measured.

## Comparison Example 2

C. H. Self reported in the Journal of Immunological Methods, vol. 76, pp. 389-393 (1985) another method of alkaline phosphatase activity detection using $NADP^+$ as the substrate in which the formazan pigment is accumulated by subjecting the resulting $NAD^+$ to cycling reaction in the presence of alcohol dehydrogenase and diaphorase to detect alkaline phosphatase with high sensitivity. The present inventors proceeded in accordance with the above-mentioned method. That is, 25 µl of 100 µM $NADP^+$ was added and reacted with alkaline phosphatase on the disc. After 1 hour, a 10 µl portion was sampled and reacted with 0.99 ml of the cycling reagent for 15 minutes; then the reaction was terminated by the addition of 1 ml of 0.3N HCl. The cycling reagent comprised 0.01 mg/ml alcohol dehydrogenase, 5% ethanol, 0.5% Triton X-100, 0.1 mg/ml diaphorase and 0.1 mg/ml nitroblue tetrazolium, and it was used in solution in a 50 mM phosphate buffer, pH 7.5.

For cycling conditions, optimum composition and concentration were used, but the blank absorbance was high, and it was thus impossible to measure the resulting $NAD^+$ with high sensitivity (measurement results are given in Table 1).

Table 1

| | Luminescence method | SNAP kit | Cycling method |
|---|---|---|---|
| Dilution series (number of copies) | $\Delta I_0$ (S/N) | $\Delta$ E (S/N) | $\Delta$ E (S/N) |
| 1 ($1.4 \times 10^9$) | 43.01 (232.3) | 44.09 (7.32) | 43.41 (2.91) |
| 1/4 ($3.5 \times 10^8$) | 12.35 (67.42) | 30.99 (5.44) | 42.81 (2.91) |
| $1/4^2$ ($8.75 \times 10^7$) | 1.86 (10.99) | 19.31 (3.77) | 33.4 (2.49) |
| $1/4^3$ ($2.2 \times 10^7$) | 0.548 (3.95) | 7.81 (2.13) | 1.68 (1.07) |
| $1/4^4$ ($5.46 \times 10^6$) | 0.325 (2.75) | 3.05 (1.44) | - |
| $1/4^5$ ($1.37 \times 10^6$) | 0.157 (1.84) | - | - |
| $1/4^6$ ($3.4 \times 10^5$) | 0.094 (1.51) | - | - |
| $1/4^7$ ($8.54 \times 10^4$) | 0.052 (1.28) | - | - |
| $1/4^8$ ($2.13 \times 10^4$) | 0.033 (1.18) | - | - |

## Comparison Example 3

Alcohol dehydrogenase and other dehydrogenase were compared in catalysis of NAD - NADH reaction. The tested enzymes, all of which were NAD-specific and readily available, were glycerol dehydrogenase (EC 1.1.1.6, bacterial derivation, Toyobo, 50 U/mg), glyceraldehyde triphosphate dehydrogenase (EC 1.2.1.12, yeast derivation, Sigma, 120 U/mg), lactic acid dehydrogenase (EC 1.1.1.6, organ derivation, Sigma, 380 U/mg), formaldehyde dehydrogenase (EC 1.2.1.-, bacterial derivation, Toyobo, 2 U/mg) and malic acid dehydrogenase (EC 1.1.1.37, organ derivation, Sigma, 480 U/mg).

An alkaline phosphatase-coupled disc prepared in the same manner as in Example 1 was subjected to luminescence reaction under the same conditions as in Example 1. Dehydrogenation conditions were settled according to enzymes as follows:

| Enzyme | Concentration | Substrate | Concentration |
|---|---|---|---|
| ADH | 2 U/ml | Ethyl alcohol | 21 mM |
| GDH | 3 U/ml | Glycerol | 20 mM |
| GA3DH | 3 U/ml | Glyceraldehyde 3-phosphate | 0.2 mM |
| LDH | 2 U/ml | Lactic acid | 5 mM |
| FDH | 8 U/ml | Formaldehyde | 0.2 mM |
| MDH | 2 U/ml | Malic acid | 1 mM |
| ADH: Alcohol dehydrogenase<br>GDH: Glycerol dehydrogenase<br>GA3DH: Glyceraldehyde 3-phosphate dehydrogenase<br>LDH: Lactic acid dehydrogenase<br>FDH: Formaldehyde dehydrogenase<br>MDH: Malic acid dehydrogenase | | | |

Luminescence was measured with 3.5 x $10^6$ copies of the plasmid of Example 1 as the sample under respective conditions. The results are shown in the table below.

| Enzyme | $\Delta I_0$ | S/N |
|---|---|---|
| ADH | 12.28 | 64.53 |
| GDH | 8.65 | 57.64 |
| GA3DH | 10.85 | 12.49 |
| LDH | 6.47 | 30.48 |
| FDH | 4.32 | 8.72 |
| MDH | 5.67 | 28.49 |

As is evident from this table, alcohol dehydrogenase is the dehydrogenase which permits luminescence assay with high reactivity and high S/N ratio.

The DNA probe detection method of the present invention is excellent in that it surpasses the absorption photometric method in sensitivity and stability because it combines a DNA probe labeled with alkaline phosphatase with a luminescent system.

Also, the use of $NADP^+$ as the substrate for alkaline phosphatase permits the obtainment of maximum activity with a small amount of substrate and in addition, offers improved luminescence detection sensitivity. The use of alcohol dehydrogenase as a means of reducing $NAD^+$ to NADH avoids adverse influence on alkaline phosphatase activity. The use of flavin reductase and luciferase as means of luminescence detection permits quick and, due to no effect on other enzyme or reaction, accurate detection of the amount of NADH as an amount of luminescence.

Another noticeable effect of the present invention is that the reaction system is not affected by the surfactant used for nucleic acid hybridization.

## Claims

1. A DNA probe assay method with a bacterial luciferase system, characterized in that a DNA probe labeled with alkaline phosphatase is reacted with $NADP^+$ and the resulting NAD. is reacted with alcohol and alcohol dehydrogenase to convert it to NADH, which is then measured as a luminescence intensity using bacterium-derived flavin reductase and luciferase.

2. An assay method as claimed in Claim 1 wherein the alcohol dehydrogenase is of yeast derivation.

3. An assay method as claimed in Claim 1 wherein the alcohol dehydrogenase is of equine liver derivation.

4. An assay method as claimed in Claim 1, characterized in that ethyl alcohol is used in converting $NAD^+$ to NADH by the reaction with alcohol dehydrogenase.

5. An assay method as claimed in Claim 1, characterized in that an amount of NADH is measured with

the use of bacterium-derived flavin reductase and luciferase in the presence of FMN and a long-chain aldehyde.

6. An assay method as claimed in Claim 1, characterized in that the bacterium-derived flavin reductase is selected from a group of Vibrio harveyi and Vibrio fischeri.